# EUROPEAN PATENT SPECIFICATION

(11) **EP 1 180 036 B1**
(45) Date of publication and mention of the grant of the patent: **07.10.2009**
(21) Application number: 00918005.0
(22) Date of filing: 16.03.2000
(51) Int. Cl.: A61K 35/30, C12N 5/06, C12N 5/08, A61P 9/00, A61P 21/00, A61P 25/00

(54) **CELL THERAPY FOR CHRONIC STROKE**
ZELLTHERAPIE FÜR CHRONISCHEN SCHLAGANFALL
CYTOTHERAPIE CONTRE L'ATTAQUE CHRONIQUE

(30) Priority: 27.04.1999 US 131230 P; 20.07.1999 US 144785 P
(43) Date of publication of application: 20.02.2002
(60) Divisional application: 08006165.8
(73) Proprietor: Layton Bioscience, Inc., Sunnyvale, CA 94086 (US); University of South Florida, Tampa, FL 33612 (US)
(72) Inventor: SANBERG, Paul, R., Spring Hill, FL 34610 (US); KONDZIOLKA, Douglas, Pittsburgh, PA 15215 (US); MCGROGAN, Michael, P., San Carlos, CA 94070 (US); SNABLE, Gary, L., Atherton, CA 94027 (US)
(74) Representative: Roques, Sarah Elizabeth
(86) International application number: PCT/US2000/006912
(87) International publication number: WO 2000/064459

(56) References cited:
- WO-A-98/57663
- US-A- 5 851 832
- LAYTONBIO: "Press releases number: 24, 26, 27, 28, 29, 30, 31(j) and (k)" WWW.LAYTONBIO.COM, [Online] 9 March 1998 (1998-03-09) - 5 February 1999 (1999-02-05), XP002199748 Retrieved from the Internet: <URL:http://www.laytonbio.com/whatsnew.htm >
- BONN-D: "First cell transplant aimed to reverse stroke damage" THE LANCET, vol. 352, 11 July 1998 (1998-07-11), page 119 XP002199789
- SIMM-M: "Nachschub für die grauen Zellen" BERLINER ONLINE, [Online] 3 March 1999 (1999-03-03), XP002199747 Retrieved from the Internet: <URL:http://www.BerlinOnline.de/.bin/print .php/wissen/berliner_zeitung/archiv/1999/0 303/wissenschaft/0002/index.html?keywords= Nachschub%20f%FCr%20die%20grauen%20Zellen% 20&ok=OK%21&match=strict&author=&ressort=W issenschaft&von=1.2.1999&bis=1.5.1999&mark =zellen%20nachschub%20die%20grauen%20fur%2 0f%FCr> [retrieved on 2002-05-21]
- FLAX-JD ET AL.: "Engraftable human neural stem cells respond to developmental cues, replace neurons, and express foreign genes" NATURE BIOTECHNOLOGY, vol. 16, November 1998 (1998-11), pages 1033-1039, XP002199749
- BORLONGAN ET AL.: 'Viability and survival of hNT neurons determine degree of functional recovery in grafted ischemic rats' NEUROREPORT, vol. 9, no. 12, 24 August 1998, pages 2837 - 2842, XP002929862
- BORLONGAN-CV: "Transplantation of cryopreserved human embryonal carcinoma-derived neurons (NT2N cells) promotes functional recovery in ischemic rats" EXPERIMENTAL NEUROLOGY, vol. 149, 1998, pages 310-321,
- KONDZIOLKA-D ET AL.: "Neural transplantation for stroke" JOURNAL OF CLINICAL NEUROSCIENCE, vol. 9, no. 3, 2002, page 225-230,
- KONDZIOLKA-D ET AL.: "Transplantation of cultured human neuronal cells for patients with stroke" NEUROLOGY, vol. 55, August 2000 (2000-08), page 565-569,
- WECHSLER-LR ET AL.: "Cell Therapy: Replacement" STROKE, vol. 34, 2003, page 2081-2082,

## Description

### Technical Field

This invention is in the medical treatment of neurological deficits resulting from stroke; more specifically, the invention applies cell therapy to restore lost cognitive, motor, sensory and speech function resulting from stroke.

### Background of the Art

In the United States, according to the National Institutes of Health, stroke is the third leading cause of death and the most common cause of adult disability. With an incidence of approximately 750,000 patients, approximately 30% (250,000) die, 30% (250,000) become severely and permanently disabled, and 30% (250,000) recover with little or no functional deficits. Currently four million Americans are living with the effects of stroke, and two thirds of those have moderate to severe impairments. In addition, improving diagnostic methods, such as diffusion-weighted imaging (showing dead brain tissue) and perfusion-weighted imaging (showing oxygen-starved but live brain tissue), are helping diagnose more new and old strokes.

Stroke is defined as a sudden, non-convulsive, focal neurologic deficit that is related either to cerebral ischemia or hemorrhage. The neurologic deficit created reflects the location and size of the cerebral infarction. Lacunar infarction is one type of ischemic stroke that is usually of small volume, and which may be typified by various clinical syndromes (e.g., hemiparesis with ataxia in the same limb, pure motor hemiplegia). When located in a region of non-critical brain tissue, lacunar stroke is often not associated with symptoms. However, when located in a critical structure such as the internal capsule, thalamus, basal ganglia or brain stem, significant neurologic disability can occur.

After a stroke has occurred, treatment in the acute setting can consist of thrombolytic therapy, surgical resection of large strokes that cause major mass effect and coma, and rare reperfusion techniques such as extracranial-intracranial bypass. Neuroprotective agents such as glutamate receptor inhibitors or inhibitors of excitatory amino acid release were in clinical trials for treatment within the first six to twelve hours of stroke onset. To date, none of these trials has been successful since it is difficult for the stroke victim to reach the hospital within the narrow (3-6 hour) window during which the neuroprotective agents can rescue damaged neuronal cells. Agents that interfere with nitric oxide synthesis or generation of free radicals have also been tested.

Once the acute phase of the incident has passed, the patient enters rehabilitation for motor and cognitive function, as required. Rehabilitation therapy is an important part of stroke management, during which many patients have significant recovery. As much as 90% of a patient's recovery occurs in the first 30 days after the stroke. Generally, the longer the delay in recovery, the poorer the prognosis. If recovery does not begin within one or two weeks, the outcome is poor for motor, sensory, speech, and cognitive function.

The concept of cellular implantation for the treatment of chronic neurologic deficits after stroke was raised in an editorial in the ANNALS OF NEUROLOGY several years ago. Brain repair through the implantation of cells, growth factors, or other neurotransmitters was postulated to represent the future of stroke management. The development of cultured human neuronal cells represents an important step in this line of research. To understand the foundations of cellular brain restoration, several concepts are important. First, we must understand the disorder, and understanding that remains variable at this time. Second, we must develop appropriate cell lines for transplantation. Third, we must develop the technologies and skills for surgery. Stereotactic techniques are well established in the neurosurgical realm. Fourth, we must establish the safety of transplantation procedures. Fifth, we must establish which cell types are appropriate for restoration of function including the number of cells and the locations of transplants. Sixth, we must define what else is required to assist cellular function such as growth factors or cellular matrices, and an appropriate course of post-implant rehabilitation. Seventh, we must define reasonable outcomes and expectations for our patients.

HNT neuronal cells were initially produced from a lung metastasis tumor removed from a 22-year-old patient with a testicular teratocarcinoma in 1972 at the Sloan Kettering Cancer Center in New York City. Dr. Peter Andrews at the Wistar Institute in Philadelphia was the first to observe that these cells exhibited the unique property of differentiating into embryonic neuronal cells upon treatment with retinoic acid. He published this observation in 1984. Dr. Virginia M.-Y. Lee, working at the University of Pennsylvania, then developed the process for producing large quantities of the human embryonic neuronal cells (U.S. Patent No. 5,175,103).

In various animal models, these cells have been shown to mature, integrate and survive for over one year in the nude mouse brain and interestingly show an intense propensity to develop processes that even cross the midline of the brain.

A number of degenerative brain disorders have been proposed for neurotransplantation. These include acute and chronic stroke, Parkinson's disease, Huntington's disease, head injury, spinal cord injury and others. No treatment now exists to restore lost brain function after stroke. We theorized that treating stroke patients by implanting suitable cells into the patients' stroked areas might lead to the cells' integration into the host brain, resulting in restoration of lost neural function.

### Summary of the Invention

We provide neuronal cells for use in treating stroke in a patient who has undergone a stroke at least three hours earlier, by a method which comprises delivering at least six million viable neuronal cells at a concentration of cells based on the total viable cell count to at least one brain area involved in the stroke, wherein if said cells are stem cells they have not been obtained by destruction of a human embryo. Optionally, the method also includes the step of using a twist drill or a burr to form a hole in the skull through which the cells could be administered. Cells for administration in the method are selected from the group consisting of hNT neuronal cells, HCN-1 cells, fetal pig cells, neural crest cells or a combination thereof.

Also disclosed is a pharmaceutical composition of 95% pure neuronal cells which is packaged in a vial with PBS. The vial is further encased in a container with liquid nitrogen, whereby the composition is kept at -170°C before use.

Also disclosed are neuronal cells for use in for improving speech in a person who has experienced brain damage which interferes with speech, wherein the brain damage results from stroke, by a method which comprises injecting a sterile composition of at least six million viable neuronal cells at a concentration of cells based on the total viable cell count into the damaged area, wherein if said cells are stem cells they have not been obtained by destruction of a human embryo. Such brain damage is due to stroke. The injected neuronal cells may be human neuronal cells.

Also disclosed are neuronal cells for use in improving motor performance in a person who has experienced brain damage which interferes with movement, wherein the brain damage results from stroke, by a method which comprises injecting a sterile composition of at least six million viable neuronal cells at a concentration of cells based on the total viable cell count to the damaged area wherein if said cells are stem cells they have not been obtained by destruction of a human embryo. Such brain damage is due to stroke. The injected cells may be human neuronal cells.

Also disclosed are neuronal cells or neural stem cells for use in improving cognition in a person who has experienced brain damage which interferes with cognition, wherein the brain damage results from stroke, by a method which comprises delivering a sterile composition of at least six million viable neuronal cells at a concentration of cells based on the total viable cell count to the damaged area of the brain, wherein if said cells are stem cells they have not been obtained by destruction of a human embryo. Such brain damage is due to stroke: The injected neuronal cells may be human neuronal cells or neural stem cells.

Also disclosed are neuronal cells for use in improving sensory function in a person who has experienced brain damage which interferes with sensation, wherein the brain damage results from stroke by a method which comprises delivering a sterile composition of at least six million viable neuronal cells at a concentration of cells based on the total viable cell count to the damaged area wherein if said cells are stem cells they have not been obtained by destruction of a human embryo.

### Brief Description of the Drawings

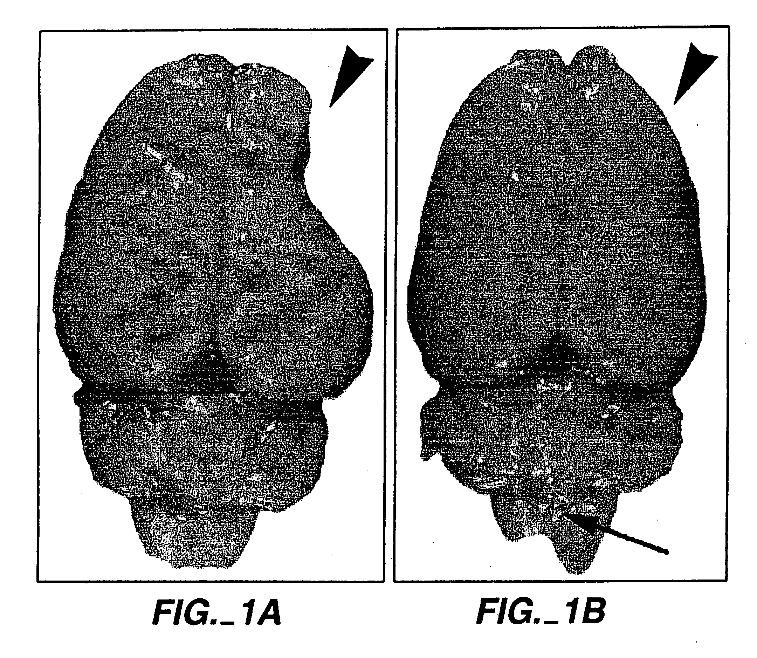

Figures 1A and 1B show rat brains subjected to middle cerebral artery infarction. Fig. 1A shows the significant infarction and loss of brain tissue in a rat treated only with vehicle; Fig. 1B shows the normal brain shape of a rat treated at three days post-infarct with a combination of neural stem cells and basic fibroblast growth factor.

### Detailed Description

To establish the utility of neuronal cell implants in patients with established stroke deficits, a study was undertaken with a randomized, open-label trial with observer-blind neurologic evaluation of patients with a cerebral infarction involving the basal ganglia region of the brain who receive stereotactic injections of hNT neuronal cells.

Substantial fixed motor deficit following stroke is a significant medical problem that needs to be better addressed. Currently, rehabilitation is the only widely practice therapy. Although fetal tissue is being utilized for the treatment of some neurologic diseases, logistical and ethical problems may hinder its widespread use for neural transplantation. The use of alternative graft sources such as LBS-Neurons and other cells (see below) is therefore appealing.

### a) hNT Neuronal Cells

hNT neuronal cells, licensed from the University of Pennsylvania, are human neuronal cells derived from a single cell line. Through eight years of *in vitro* and *in vivo* preclinical testing, the cells have been demonstrated to be human, fully post-mitotic, non-tumorigenic neuronal cells which demonstrate efficacy in animal models. After safety studies were performed in mice, rats and primates, implantation of human neurons into rats with basal ganglia stroke showed both motor and behavioral recovery in comparison to sham controls. A second experiment shows that the number of cells implanted correlated with the degree of recovery. The first clinical study evaluated the product as a somatic cell therapy that produced a novel way to restore lost cognitive and motor function. Further, early research is being planned in the use of hNT neuronal cells as a platform for the introduction and expression of specific human neuronal genes into the brain for the treatment of neurologic disorders.

HNT neuronal cells were derived by treating the neuronal precursor cell line NT2/D 1 derived from an embryonic carcinoma with retinoic acid and mitotic inhibitors. Following treatment with retinoic acid, the NT2/D1 cells differentiate into non-proliferating, terminally differentiated neurons and proliferating non-neuronal accessory cells (Andrews, P.W. Dev. Biol. 103:285-293, 1984).

After subsequent treatment with mitotic inhibitors (cytosine arabinoside and fluorodeoxyuridine), pure cultures of post-mitotic human neuronal cells result (Pleasure and Lee, 1993). These cells were then suspended in freezing medium (HAS, DMSO and PBS) and frozen in ampoules. The resultant product, when produced in compliance with current Good Manufacturing Practice (cGMP) guidelines, is called LBS-Neurons human neuronal cells.

The NT2/D1 cell line was established in culture as a cell line by Dr. Peter Andrews at the Wistar Institute in Philadelphia during the early 1980s. Dr. Andrews received the original cells (known as Tera-2) from Dr. Jurgen Fögh of the Sloan Kettering Institute in New York City. The Tera-2 cells had been isolated from a pulmonary embryonic carcinoma of a 22-year-old Caucasian male with a metastasized primary testicular germ cell tumor.

The post-mitotic human neuronal cells available as hNT neuronal cells resulted from the differentiation of NT2/D1 cells in response to retinoic acid. These human neuronal cells actively demonstrate neurite outgrowth, sending out numerous processes that assemble into neuronal networks. They also form polarized processes that have been identified functionally as axons and dendrites, and demonstrate the ability to form synapses upon maturation. These cells have retained their human characteristics as demonstrated by isoenzyme typing, expression of a variety of human antigens, and by karyotyping (Andrews et al., *ibid.,* Miyazono et al., 1996, Layton Bioscience, Inc., 1996).

Furthermore, hNT cells have been successfully implanted in various animal models where they histologically integrated with the neurons and sent processes into adjacent tissue. A recent report describes the results of transplanting hNT cells in rats with sustained ischemic damage. Transplants of 0, 5, 10, 20, 40, 80 or 160x10³ neurons produced dose-dependent improvement in function and hNT survival. Animals receiving 40, 80 or 160x10³ neurons produced a dose-dependent improvement in both passive avoidance and elevated body swing tests. Transplants of 80 or 160x10³ hNT neurons demonstrated a 12-15% survival of hNT neurons in the graft, while transplants of 40x10³ hNT neurons resulted in a 5% survival.

Moreover, similar improvement was seen in rats with cerebral ischemia induced by occlusion of the middle cerebral artery. The viability and survival of hNT neurons were evaluated before transplantation and at three month after transplantation in ischemic rats. Monthly behavioral tests (1, 2 and 3 months after implant) showed that ischemic animals receiving intrastriatal implants (about 4x10 cells) displayed normalization of asymmetrical motor behavior compared with ischemic animals that received medium alone. Within-subject comparisons of cell viability and subsequent behavioral changes revealed that a high cell viability just prior to transplantation surgery correlated highly with a robust and sustained functional improvement in the transplant recipient. There also was a positive correlation between the number of surviving hNT neurons and the degree of functional recovery (Borlongan CV et al. Neuroreport 9(12): 2837-42, 1998).

### b) Other cells

Other cells may be used in the transplant procedures disclosed herein, provided they meet the following criteria: Non-immunogenic, non-tumorigenic, reproducible, adapting to the transplant location and synapsing with the local neurons. The following are only a few examples of cells that could be readily tested according to the procedures given in this patent application.

The HCN-1 cell line is derived from parental cell lines from the cortical tissue of a patient with unilateral megalencephaly growth (Ronnett G.V. et al. Science 248:603-5, 1990). HCN-1A cells have been induced to differentiate to a neuronal-like morphology and stain positively for neurofilament, neuron-specific enolase and p75NGFR, but not for myelin basic protein, S-100 or glial fibrillary acidic protein (GFAP). Because these cells also stain positively for γ-amino butyric acid and glutamate, they appear to become neuro-transmitting bodies. Earlier Poltorak M et al. (Cell Transplant 1(1):3-15, 1992) observed that HCN-1 cells survived in the brain parenchyma and proposed that these cells may be suitable for intracerebral transplantation in humans. Ronnet GV et al. (Neuroscience 63(4):1081-99, 1994) reported that HCN-1 cells grew processes resembling neurons when exposed to nerve growth factor, dibutyryl cyclic AMP and isobutylmethylxanthine.

The nerve cells also can be administered with macrophages that have been activated by exposure to peripheral nerve cells. Such activated macrophages have been shown to clean up the site of CNS trauma, for example, a severed optic nerve, after which new nerve extensions started to grow across the lesion. Implanting macrophages exposed to CNS tissue (which secretes a chemical to inhibit macrophages) or nothing at all resulted in little or no regeneration (Lazarov-Spiegler et al. FASEB J. 10:1, 1996).

Sertoli cells have been disclosed in U.S. Patent No. 5,830,460 to University of South Florida as producing a sustained localized brain immunosuppressive effect on transplantation into the brain tissue. Hybrid Sertoli-secretory cells disclosed in U.S. Patent 5,827,736 also can be useful in the present invention, where the stroke destroys secretory cells.

U.S. Patent No. 5,753,505 to Emory University discloses a cellular composition which is greater than about 90% mammalian, non-tumor-derived, neuronal progenitor cells which express a neuron-specific marker and which can give rise to progeny which can differentiate into neuronal cells. The cells are proposed for treatment of neuronal disorders.

U.S. Patent No. 5,753,491 discloses human fetal neuro-derived cells lines as well as method of implanting the immortalized cells into a host. The cells are provided with a heterologous nucleic acid for a biologically active peptide, such as tyrosine hydroxylase. The cells may be delivered with other cells, such as hNT cells or PC12 cells.

Gage et al. (U.S. Patent No. 5,766,948 and others) has disclosed methods for producing a neuroblast and a cellular composition which is an enriched population of neuroblast cells. These cells can be used to treat neuronal disorders.

Fetal pig cells have been implanted into patients with neurodegenerative diseases,' such as Parkinson's disease and Huntington's chorea, and intractable seizures, in whom surgical removal of the excited area would otherwise have been performed. Such cells, if properly screened for retroviruses, could also be used in the inventive method.

Neural cells with stem cell properties have been isolated by Snyder et al., from the human fetal brain and propagated *in vitro* by a variety of equally effective and safe means - both epigenetic (e.g., with mitogens such as epidermal growth factor (EGF) or basic fibroblast growth factor (bFGF) or with membrane substrates) and genetic (e.g., with propagating genes such as *vmyc* or large T-antigen) (Flax, JD et al., Nature Biotechnology 16:1033-39, 1998).

Murine neural stem cells (NSCs) were recently administered to adult rats whose middle cerebral artery (MCA) was obstructed to produce experimental and dramatic cerebral tissue loss (see Figure 1A). Fig. 1A shows an infarcted rat brain, into which vehicle alone had been injected intracerebrally. The large infarct cavity (white arrowhead) represents significant tissue loss. Fig. 1B is a photo of a rat brain subjected to a similar infarct but treated three days later with a cisternal (region indicated by black arrow) infusion of a cellular suspension of murine NSCs plus basic fibroblast growth factor (bFGF), which is a significant distance from the region of infarction. Nevertheless, the NSCs appear to have migrated to the region of damage and significantly ameliorated the cerebral volume loss (white arrowhead), appearing to have helped "fill in" the infarction cavity and reverse the tissue loss. In preliminary studies, animals treated in this manner showed a significant improvement in cortically mediated behavioral tasks. Therefore, these results indicate that NSC were drawn to stroke injuries in adult CNS.

### c) Other cytokines, growth factors and drugs

It may be beneficial to administer certain cytokines, growth factors and drugs in the transplant area. Such moieties are optionally used or may be administered concomitantly with the transplant or later.

Known cytokines include interleukins (IL) IL-1, IL-2, IL-3, IL-4, IL-5, IL-6, IL-7, IL-8, IL-10, and IL-11; tissue necrosis factors (TNF), TNFα and TNFβ, also lymphotoxin (LT); interferons (IFN) IFNα, IFNβ and IFNγ; tissue growth factor (TGF); and basic fibroblast growth factor (bFGF). The colony-stimulating factors (CSFs) are specific glycoproteins that are thought to be involved in the production, differentiation and function of stem cells.

Nerve growth factor (NGF) has been shown to increase the rate of recovery in spatial alternation tasks after entorhinal lesions, possibly by acting on cholinergic pathways (Stein and Will, Brain Res. 261:127-31,1983).

In addition, cyclosporine was used for at least part of the pre- and post-implant period and other similarly active compounds could be substituted. Cyclosporine was withdrawn in on patient because of seizures, and no marked diminution in function occurred thereafter. Therefore, immunosuppressive therapy may not be necessary, or perhaps could be confined only to the perioperative period.

### Other Uses

### Description of Testing Procedures

### MRI and FDG PET Scan

Observer-blinded determination of neurologic status was performed, including evaluation of the functional deficit, contrast-enhanced magnetic resonance image (MRI) scanning to measure the volume of blood-brain barrier alteration at the target site (as an indirect measure of inflammatory response), and positron emission tomography (PET) with fluorodeoxyglucose (FDG) scan for assessment of regional brain metabolism.

### NIH Stroke Scale

This procedure was modified from that of Brott T, Adams HP, Olinger CP, et al. (1989) Measurements of acute cerebral infarction: a clinical examination scale. *Stroke* 20:864-870. Stroke scale items were administered in the order listed below. Performance was recorded in each category after each subscale exam. Personnel were forbidden from going back and changing scores. Specific directions were provided for each exam technique.

### European Stroke Scale

This procedure was adapted from that reported in Hantson L, De Weerdt W, De Keyser J, et al. (1994) The European Stroke Scale. Stroke 25:2215-2219.

### I. LEVEL OF CONSCIOUSNESS

A score of 10 is assigned alert, keenly responsive patients; a score of 8 to drowsy patients who can be aroused by minor stimulation to obey, answer or respond; a score of 6 to patients who require repeated stimulation to attend, or are lethargic or obtunded and require strong or painful stimulation to move; a score of 4 to patients who cannot be aroused by any stimulation but react purposefully to painful stimuli; a score of 2 to patients who cannot be aroused by any stimulation and react decerebrately to painful stimuli; and a score of 0 to patients who cannot be aroused by any stimulation and do not react to painful stimuli.

### II. COMPREHENSION

The examiner, without demonstrating, verbally gives the patient the following commands: 1. Stick out your tongue. 2. Put your finger (of the unaffected side) on your nose. 3. Close your eyes.

### III. SPEECH

The examiner has a conversation with the patient (how is the patient feeling, did he/she sleep well, how long has the patient been in the hospital...) and scores the patient as follows: normal speech (8), slight word-finding difficult but possible conversation (6), severe word-finding difficulties with difficult conversation (4), only yes or no (2), and mute (0).

### IV. VISUAL FIELD

The examiner stands at arm's length and compares the patient's field of vision by advancing a moving finger from the periphery inward. The patient fixates on the examiner's pupil, first with one and then with the other eye closed. Normal is 8 and deficit is 0.

### V. GAZE

The examiner steadies the patient's head and asks him/her to follow the examiner's moving finger. The examiner observes the resting eye position and subsequently the full range of movements by moving the index finger from the left to the right and back. Normal is 8, median eye position with impossible deviation to one side (4), lateral eye position with possible return to midline (2), and lateral eye position without return to midline (0).

### VI. FACIAL MOVEMENT

The examiner observes the patient as he/she talks and smiles, noting any asymmetrical elevation of one corner of the mouth or flattening of the nasolabial fold. Only the muscles of the lower half of the face are assessed. Normal is 8, paresis 4, and paralysis 0.

### VII. ARM (maintain outstretched position)

The examiner asks the patient to close his/her eyes and actively lifts the patient's arms into position so that they are outstretched at 45° in relation to the horizontal plane with both hands in mid-position so that the palms face each other. The patient is asked to maintain this position for 5 seconds after the examiner releases the arms. Only the affected side is evaluated. Score is 4 for maintaining arm position for 5 sec; 3 is maintaining position for 5 sec with hand pronation; score is 2 if arm drifts before 5 sec and maintains a lower position; score is 1 if arm cannot maintain position but attempts to oppose gravity; and 0 if arm falls.

### VIII. ARM (raising)

The patient's arm is rested next to the leg with the hand in mid-position. The examiner asks the patient to raise the arm outstretched to 90° (4), if the arm is straight but movement is not full (3), flexed arm (2), trace movements (1), or no movement (0).

### IX. EXTENSION OF THE WRIST

The patient is tested with the forearm supported and the hand unsupported, relaxed in pronation. The patient is asked to extend the hand. Normal, fully isolated movement with no decrease in strength is 8, full isolated movement with reduced strength is 6, movement not isolated and/or full is 4, trace movement is 2, and no movement is 0.

### X. FINGERS

The examiner asks the patient to form with both hands, as strongly as possible, a pinch grip with the thumb and forefinger on the same hand and to try to resist a weak pull. The examiner checks the strength of this grip by pulling the pinch with one finger. Equal strength is 8, reduced strength on the affected side is 4, and pinch grip impossible on affected side is 0.

### XI. LEG (maintain position)

The examiner actively lifts the patient's affected leg into position so that the thigh forms an angle of 90° with the bed. The examiner asks the patient to close his/her eyes and to maintain this position for 5 seconds without support. Leg maintains position for 5 sec (4), leg drifts to intermediate position by 5 sec (2), leg drifts to bed within 5 sec but not immediately (1), and leg falls to bed immediately (0).

### XII. LEG (flexing)

The patient is supine with the legs outstretched. The examiner asks the patient to flex the hip and knee. Normal movement is 4, movement against resistance with reduced strength is 3, movement against gravity is 2, trace movement is 1, and no movement is 0.

### XIII. DORSIFLEXION OF THE FOOT

The patient is tested with the leg outstretched. The examiner asks the patient to dorsiflex the foot. Normal (e.g., outstretched, full movement, normal strength) is 8, leg outstretched with full movement but reduced strength is 6, leg outstretched with less than full movement or flexed knee or supinated foot is 4, trace movement is 2, and no movement is 0.

### XIV. GAIT

A normal gait scores 10, gait with abnormal aspect and/or limited distance or speed is 8, walking with aid is 6, requiring the assistance of one or more persons is 4, no walking but standing supported is 2, and no walking or standing is 0.

### Barthel Index

This test has been modified from that described in Mahoney FI, Barthel DW. (1965 Functional evaluation: the Barthel Index. Md State Med J 14:61-65). It includes a number of life activities, including feeding getting out of and returning to bed, toilet activities, walking, handling stairs, dressing, controlling bowel and bladder.

### SF-36 Health Survey

This survey has been modified from Ware JE, Sherbourne CD. (1992) The MOS 36-item short-form health survey (SF-36). 1. Conceptual framework and item selection. Med Care 30:473-483. It includes general health, comparison to a year earlier, competence at daily activities, ability to work, and emotional status,

### CLINICAL EXAMPLES

Patients with stable strokes and fixed deficits were recruited for a Phase I safety trial. Inclusion criteria included major motor deficit from completed basal ganglia stroke defined on imaging. The permissible duration of stroke was six months to six years, with a required fixed deficit without substantial change for at least two months. Patient age could range from 40 to 75 years inclusive. The patient also had to be able to provide informed consent. Patients must have had a motor deficit such as hemiparesis following a completed basal ganglia infarction (4-15 mm) involving gray matter as defined on CT or MR imaging scan and by clinical syndromes of lacunar infarction (e.g., hemiparesis with ataxia in the same limb, pure motor hemiplegia). A substantial deficit was defined by a total score of 70 or less on the European Stroke Scale (see infra).

Preoperative investigations included serial stroke scales (three) over two months prior to surgery. Imaging studies included MRI scan, FDG-PET studies as well as functional MRI. Quality of life scales with the Barthel index and the SF36 as well as serologic tests and video taping were performed. Postoperative investigations included clinical assessments and stroke scales at regular intervals over the first year with serologic tests, MRI scans and research MRI scans as well as PET scans at six and 12 months.

For immunosuppression patients received 6 mg/kg of Cyclosporine-A per day, administered orally once daily. However, the dose was adjusted according to the results of serum levels. The drug was administered beginning one week prior to surgery and continued for eight weeks after surgery. Methylprednisolone (40 mg IV) also was administered during surgery.

Prohibited medications (for at least 1 week prior to surgery) were all products with anticoagulant or anti-platelet activity, including warfarin, aspirin, nonsteroidal antiinflammatory drugs (NSAIDs), and ticlopidine. These medications were allowed to be restarted 24 hours after surgery.

On the morning of surgery, cells were prepared for implantation. One ml frozen LBS-Neurons cryoampules had been filled with a suspension containing 6.0x10⁶ human neuronal cells per ml. It is important to thaw the neurons no more than one hour prior to use, because their viability begins to decrease after 2 hours on ice in phosphate buffer solution. It takes approximately 30-45 minutes to prepare the cells for injection. The cryopreserved suspension stored frozen at -170 °C, thawed rapidly in a 37° C water bath with gentle agitation until the contents were just liquefied. The suspension was gently mixed to resuspend the cells.

To maintain sterile conditions, gowned and gloved personnel performed the ensuing steps under a hood. The thawed cell suspension was transferred from the cryovials to sterile 15 mL centrifuge tubes containing Isolyte® S, pH 7.4 (multi-electrolyte injection, McGaw Inc., Irvine, CA), centrifuged at 200xg for 7 minutes at room temperature and the cell pellet gently resuspended in Isolyte S. This wash of the cells was repeated twice. For the final wash, all cells from different tubes were pooled together into one tube. Next a sample of the LBS-neuron suspension was diluted in 0.4% Trypan blue, and viable and dead cells counted using phase contrast microscopy. The cell concentration was calculated based on the total viable cell count. The pellet volume was measured, and the cells resuspended to a final concentration of 3.3x10⁷ cells/mL in Isolyte S and aliquoted at 120 µL per sterile 1.0 mL vial. Depending on the dose to be administered, one or more vials were prepared. Vial(s) were loaded into a closed holder and carried by hand in an upright position to the operating room for immediate use.

The cells were administered (in up to three tracts) by direct stereotactic injection. The first four patients received two million cells in three implants on one track, and the next eight patients where randomized to receive two or six million cells in three or nine implants, respectively. Aliquots of cells that were placed in culture and not implanted showed robust development of neuronal processes with 24 hours. Patients stopped all anticoagulant medications and started cyclosporine one week prior to surgery.

Surgery began with stereotactic frame application under local anesthesia and mild sedation. Stereotactic instrumentation consisted of the following: Leksell Model G Stereotactic Coordinate Frame (Elekta Instruments, Atlanta, GA) and a 0.9 mm Outer Diameter Stereotactic Aspiration/Injection Cannula. Contrast-enhanced computed tomography (CT) stereotactic targeting of the stroke area was performed with 5-millimeter slices through the brain. Coronal and sagittal views were used to define a safe trajectory that entered a cortical gyrus and spared a sulcus. Stereotactic coordinates were obtained for each instrument placement. Three points in the basal ganglia were a) inferior to the stroke, b) within the midportion of the stroke, and c) in the superior aspect of the basal ganglia either within or beyond the stroke. For patients receiving nine implants (6x10⁶ cells), three trajectories were chosen in the same paramedian plane, spaced by 5-6 mm at the target. A twist drill or burr hole skull opening was made. The dura was opened and a 1.8-mm, 15-cm length stabilizing probe inserted to a point 4 cm proximal to the final target. A cannula with a 0.9-mm outer diameter was then inserted down to the deepest target point for the first implantation. The first inner cannula used had an internal volume of 100µL; a second cannula designed later had a volume of 20 µL (Synergetics, St. Louis, MO). In the operating room, the cells were aspirated into a 250 µl syringe. The internal volume of the cannula was filled with the cell suspension, and then a 20 µl volume of cells was injected slowly at the first target site. The instrument was then withdrawn to the second and third sites for subsequent implants. After the three implants were made, the cannula was withdrawn from the brain. The wound was either closed or the next vial of cells prepared to inject implants 4-9 in those patients who received 6x10⁶ cells. Following surgery, a post-operative CT scan confirmed the absence of hemorrhage.

A postoperative CT scan confirmed the safety of the procedure. All patients were then observed overnight and discharged home the next morning. No new neurological deficits were identified acutely. All 12 patients were discharged within 24 hours.

Follow-up assessments for safety and efficacy were made at 1 week, 1 month, 2 months, 3 months, 6 months, and then yearly (beginning with the 12 month visit) including an observer-blind neurologic examination for evaluation of the functional deficit and safety (including adverse events and follow-up laboratory tests). Contrast-enhanced MR imaging was used to measure the volume of blood brain barrier alteration at the target site and PET scanning was used for assessment of regional brain metabolism.

By the end of the study, nine male patients and three female patients had been admitted and received implants. Their age range was 44 to 75 years. The age of the stroke varied from seven months to 55 months. All strokes were confirmed to be in the basal ganglia location, and cells were placed only in that location. Four patients had involvement of adjacent cerebral cortex.

### Efficacy

Measures of efficacy were scores on the European Stroke Scale (ESS), National Institutes of Health Stroke Scale (NIHSS), Barthel Index (BI) and Short Form 36 Health Survey (SF-36) collected pre-operatively, on the day of surgery (baseline) and at predetermined intervals through 12 months following implantation of LBS-Neurons. Higher scores on the ESS, BI and SF-36 indicate better performance, and lower scores on the NIHSS indicate better performance. For this report, 6-months post-implantation was the primary time point analyzed. At 6 months following implantation, 6 of the 12 patients treated (50%) had scores on the ESS that were higher than baseline (range: 3 to 10 points), 3 patients were unchanged and 3 patients deteriorated (range: -1 to -3 points) compared to their baseline scores. Five patients (42%) had an improvement of at least 5 points on the ESS. The mean change in ESS score from baseline to week 24 for all implanted patients was 2.2 points, a difference that was statistically significant (*P* = 0.05). In the group of patients who received 2 million cells, 3 of 8 patients improved from baseline to week 24 (range: 3 to 8 points), 3 patients were unchanged, and 2 patients deteriorated (range: -1 to -3 points). In the 6-million dose group, 3 of 4 patients improved (range, 5 to 10 points) and one patient worsened (-2 points). The mean change from baseline to week 24 was 1.8 points in the 2-million group and 5.3 points in the 6-million group. The change within each treatment group was not statistically significant (*P* ≥ 0.139). NIHSS scores reflected similar changes in functional performance as seen on the ESS. At the 6-month follow-up evaluation, 8 patients had improved scores on the NIHSS (range: -1 to -4 points), 1 patient was unchanged and 3 patients deteriorated (range: 1 to 2 points) compared to their baseline scores. In the 2-million group, 5 of 8 patients improved from baseline to week 24 (range: -1 to -4 points) and in the 6-million dose group, 3 of 4 patients improved (-1 point each). The mean change in NIHSS score from week 0 to week 24 was -0.5 points for the 2-million group and -0.3 for the 6-million group. Changes from baseline on the NIHSS were not statistically significant. The BI and SF-36 did not detect substantial change in patient function.

Motor elements of the ESS (ESS-motor) accounted for the majority of the change noted in patients treated with hNT neurons. The mean change in ESS-motor score for all patients treated with hNT neurons was 2.5 (*P* = 0.026). Four patients (33%) had a change of at least 6 points on the ESS-Motor. By dose group, the mean change in ESS-motor score was 1.9 for the 2-million group (*P* = 0.186) and 3.8 for the 6-million group (P = 0.080).

PET scans performed at baseline and at week 24 showed that 6 of 11 patients had and improvement in cerebral glucose metabolism as indicated by fluorodeoxyglucose (FDG) uptake. One patient (#012) had not had a week-24 PET scan at the time of this report. The PET scan findings appeared to correlate with the clinical findings of neurologic improvement. Of the 6 patients with an increase in FDG uptake of at least 15%, 4 (67%) patients improved 3 points or more on the ESS from baseline to week 24, and 2 patients (33%) were essentially unchanged (0 and -1 point change). Of those patients with less than 15% increase in FDG uptake, 4 of 5 (80%) did not improve on the ESS and 1 patient improved by 5 points.

### Safety

There were no deaths, treatment-related serious adverse events, or early withdrawals due to adverse events. The majority of adverse events were considered mild; and the most common adverse events were fatigue, headache, nausea, and urinary tract infection. Events that were considered severe included constipation, exacerbation of chronic renal failure, increased creatinine, vomiting and dehydration, urinary tract infection, and kidney stones. There were several adverse events that were considered probably related to treatment; and all were common surgical adverse events such as headache, nausea, vomiting, blood loss with removal of the stereotactic frame and pain at the surgical site. Four patients had serious adverse events, none of which was considered by the investigator to be related to implantation of hNT neurons. One patient with diabetes had an exacerbation of his chronic renal failure while on cyclosporine, one patient had a single seizure 5 months after implantation, and one patient at 6 months after implantation had a new right pontine infarction that was contralateral to the implantation site.

No clinically significant laboratory, radiographic, or electrocardiographic abnormalities were identified that could be attributed to the hNT neurons. Cyclosporine immunosuppression was well tolerated except by one patient whose baseline serum creatinine should have excluded him from the study. Serum measures of immunologic reaction showed only minor changes that may have been indicative of a mild inflammatory reaction related to the surgical procedure itself. Serial MRI scans did not show evidence of substantial edema, inflammation, or breakdown of the blood brain barrier within or adjacent to the site of implantation. Systolic blood pressure was moderately reduced post-implantation in the 2-million cell group, but not in the 6-million cell group, and diastolic blood pressure and heart rate were not appreciably affected. None of the vital sign changes was statistically significant.

### Conclusions

The results of this study demonstrate that it is possible to safely implant hNT neurons into the basal ganglia of patients with strokes, and that these cells do not elicit an immunologic or toxic reaction within the CNS or systemically. Although the small number of patients treated precludes definitive conclusions, the stroke scale results suggest that these cells may be efficacious and that the higher dose administered may be more efficacious than the lower dose. The feasibility and preliminary safety data from this study provide the basis for the design and conduct of additional clinical trials with LBS-Neurons.

## Claims

1. Neuronal cells for use in a method of treating stroke in a patient who has undergone a stroke at least three hours earlier, the method comprising delivering at least six million viable neuronal cells at a concentration of cells based on the total viable cell count to at least one brain area involved in the stroke, wherein if said cells are stem cells they have not been obtained by destruction of a human embryo.

2. Neuronal cells according to claim 1 wherein the method further comprises using a twist drill or a burr to provide entry through the skull whereby the cells can be delivered.

3. Neuronal cells according to claim 1 or 2 wherein the cells are selected from hNT neuronal cells, neural stem cells, HCN-1 cells, fetal pig cells, neural crest cells or a combination thereof.

4. Neuronal cells according to any one of the preceding claims wherein the stroke has taken place at least three months earlier.

5. Neuronal cells for use in a method of improving speech in a person who has experienced brain damage which interferes with speech, wherein the brain damage results from stroke, the method comprising injecting a sterile composition of a at least six million viable neuronal cells at a concentration of cells based on the total viable cell count into the damaged area, wherein if said cells are stem cells they have not been obtained by destruction of a human embryo.

6. Neuronal cells according to claim 5 wherein the injected neuronal cells are human neuronal cells or human neural stem cells.

7. Neuronal cells for use in a method of improving motor performance in a person who has experienced brain damage which interferes with movement, wherein the brain damage results from stroke, the method comprising injecting a sterile composition of at least six million viable neuronal cells at a concentration of cells based on the total viable cell count to the damaged area, wherein if said cells are stem cells they have not been obtained by destruction of a human embryo.

8. Neuronal cells according to claim 7 wherein the injected neuronal cells are human neuronal cells or neural stem cells.

9. Neuronal cells for use in a method of improving cognition in a person who has experienced brain damage which interferes with cognition, wherein the brain damage results from stroke, the method comprising delivering a sterile composition of at least six million viable neuronal cells at a concentration of cells based on the total viable cell count to the damaged area of the brain, wherein if said cells are stem cells they have not been obtained by destruction of a human embryo.

10. Neuronal cells for use in a method of improving sensory function in a person who has experienced brain damage which interferes with sensation, wherein the brain damage results from stroke, the method comprising delivering a sterile composition of at least six million viable neuronal cells at a concentration of cells based on the total viable cell count to the damaged area, wherein if said cells are stem cells they have not been obtained by destruction of a human embryo.

11. Neuronal cells for use in a method of improving sensory, motor or cognitive function in a person who has experienced brain damage which interferes with those functions, wherein the brain damage results from stroke, the method comprising delivering a sterile composition of at least six million viable neuronal cells at a concentration of cells based on the total viable cell count to a location from which the neuronal cells migrate to the damaged area, wherein if said cells are stem cells they have not been obtained by destruction of a human embryo.

12. Neuronal cells according to claim 11 wherein the composition is delivered to the cisternae.

13. Neuronal cells for use in a method replacing in an individual central nervous system nerves lost to stroke, the method comprising administering to the individual a sterile composition of at least six million viable neuronal cells, at a concentration of cells based on the total viable cell count, wherein if said cells are stem cells they have not been obtained by destruction of a human embryo.

14. Neuronal cells according to any one of claims 9 to 13, wherein the neuronal cells are human neuronal cells or neural stem cells.

15. Use of neuronal cells for the manufacture of a medicament for treating stroke in a patient who has undergone a stroke at least three hours earlier, by a method which comprises delivery of at least six million viable neuronal cells at a concentration of cells based on the total viable cell count to at least one brain area involved in the stroke wherein if said cells are stem cells they have not been obtained by destruction of a human embryo.

## Patentansprüche

1. Neuronale Zellen zur Verwendung in einem Verfahren zur Behandlung eines Schlaganfalls bei einem Patienten, der mindestens drei Stunden zuvor einen Schlaganfall erlitten hat, wobei das Verfahren die Zuführung von mindestens sechs Millionen lebensfähiger neuronaler Zellen in einer auf der Gesamtzahl lebensfähiger Zellen basierenden Zellkonzentration in mindestens einen vom Schlaganfall betroffenen Hirnbereich umfasst, wobei, wenn die Zellen Stammzellen sind, diese nicht durch die Zerstörung eines menschlichen Embryos erhalten wurden.

2. Neuronale Zellen nach Anspruch 1, wobei das Verfahren des Weiteren die Verwendung eines Spiralbohrers oder einer Fräse umfasst, um Zugang durch den Schädel zu erhalten, wodurch die Zellen zugeführt werden können.

3. Neuronale Zellen nach Anspruch 1 oder 2, wobei die Zellen ausgewählt sind aus neuronalen hNT-Zellen, neuralen Stammzellen, HCN-1-Zellen, fötalen Schweinezellen, Zellen der Neuralleiste oder einer Kombination daraus.

4. Neuronale Zellen nach einem der vorstehenden Ansprüche, wobei der Schlaganfall mindestens drei Monate zuvor stattgefunden hat.

5. Neuronale Zellen zur Verwendung in einem Verfahren zur Verbesserung der Sprachfähigkeit eines Individuums, das einen Hirnschaden erlitten hat, der die Sprachfähigkeit beeinträchtigt, wobei der Hirnschaden das Ergebnis eines Schlaganfalls ist und das Verfahren das Injizieren einer sterilen Zusammensetzung von mindestens sechs Millionen lebensfähiger neuronaler Zellen in einer auf der Gesamtzahl lebensfähiger Zellen basierenden Zellkonzentration in den geschädigten Bereich umfasst, wobei, wenn die Zellen Stammzellen sind, diese nicht durch die Zerstörung eines menschlichen Embryos erhalten wurden.

6. Neuronale Zellen nach Anspruch 5, wobei die injizierten neuronalen Zellen menschliche neuronale Zellen oder menschliche neurale Stammzellen sind.

7. Neuronale Zellen zur Verwendung in einem Verfahren zur Verbesserung der motorischen Leistung bei einem Individuum, das einen Hirnschaden erlitten hat, der die Motorik beeinträchtigt, wobei der Hirnschaden das Ergebnis eines Schlaganfalls ist und das Verfahren das Injizieren einer sterilen Zusammensetzung von mindestens sechs Millionen lebensfähiger neuronaler Zellen in einer auf der Gesamtzahl lebensfähiger Zellen basierenden Zellkonzentration in den geschädigten Bereich umfasst, wobei, wenn die Zellen Stammzellen sind, diese nicht durch die Zerstörung eines menschlichen Embryos erhalten wurden.

8. Neuronale Zellen nach Anspruch 7, wobei die injizierten neuronalen Zellen neuronale Zellen oder menschliche neurale Stammzellen sind.

9. Neuronale Zellen zur Verwendung in einem Verfahren zur Verbesserung der kognitiven Leistung bei einem Individuum, das das einen Hirnschaden erlitten hat, der die kognitive Leistung beeinträchtigt, wobei der Hirnschaden das Ergebnis eines Schlaganfalls ist und das Verfahren das Zuführen einer sterilen Zusammensetzung von mindestens sechs Millionen lebensfähiger neuronaler Zellen in einer auf der Gesamtzahl lebensfähiger Zellen basierenden Zellkonzentration in den geschädigten Hirnbereich umfasst, wobei, wenn die Zellen Stammzellen sind, diese nicht durch die Zerstörung eines menschlichen Embryos erhalten wurden.

10. Neuronale Zellen zur Verwendung in einem Verfahren zur Verbesserung der sensorischen Funktion bei einem Individuum, das das einen Hirnschaden erlitten hat, der die sensorische Funktion beeinträchtigt, wobei der Hirnschaden das Ergebnis eines Schlaganfalls ist und das Verfahren das Zuführen einer sterilen Zusammensetzung von mindestens sechs Millionen lebensfähiger neuronaler Zellen in einer auf der Gesamtzahl lebensfähiger Zellen basierenden Zellkonzentration in den geschädigten Bereich umfasst, wobei, wenn die Zellen Stammzellen sind, diese nicht durch die Zerstörung eines menschlichen Embryos erhalten wurden.

11. Neuronale Zellen zur Verwendung in einem Verfahren zur Verbesserung der sensorischen, motorischen oder kognitiven Funktion bei einem Individuum, das einen Hirnschaden erlitten hat, der diese Funktionen beeinträchtigt, wobei der Hirnschaden das Ergebnis eines Schlaganfalls ist und das Verfahren das Zuführen einer sterilen Zusammensetzung von mindestens sechs Millionen lebensfähiger neuronaler Zellen in einer auf der Gesamtzahl lebensfähiger Zellen basierenden Zellkonzentration in einen Bereich umfasst, von dem aus die neuronalen Zellen zu dem geschädigten Bereich wandern, wobei, wenn die Zellen Stammzellen sind, diese nicht durch die Zerstörung eines menschlichen Embryos erhalten wurden.

12. Neuronale Zellen nach Anspruch 11, wobei die Zusammensetzung der Cisterna zugeführt wird.

13. Neuronale Zellen zur Verwendung in einem Verfahren, bei dem im Zentralnervensystem eines Individuums durch Schlaganfall verlorene Nerven ersetzt werden, wobei das Verfahren die Verabreichung einer sterilen Zusammensetzung von mindestens sechs Millionen lebensfähiger neuronaler Zellen in einer auf der Gesamtzahl lebensfähiger Zellen basierenden Zellkonzentration an das Individuum umfasst, wobei, wenn die Zellen Stammzellen sind, diese nicht durch die Zerstörung eines menschlichen Embryos erhalten wurden.

14. Neuronale Zellen nach einem der Ansprüche 9 bis 13, wobei die neuronalen Zellen menschliche neuronale Zellen oder neurale Stammzellen sind.

15. Verwendung von neuronalen Zellen für die Herstellung eines Medikaments zur Behandlung eines Schlaganfalls bei einem Patienten, der mindestens drei Stunden zuvor einen Schlaganfall erlitten hat, mithilfe eines Verfahrens, das die Zuführung von mindestens sechs Millionen lebensfähiger neuronaler Zellen in einer auf der Gesamtzahl lebensfähiger Zellen basierenden Zellkonzentration in mindestens einen vom Schlaganfall betroffenen Hirnbereich umfasst, wobei, wenn die Zellen Stammzellen sind, diese nicht durch die Zerstörung eines menschlichen Embryos erhalten wurden.

## Revendications

1. Cellules neuronales à utiliser dans un procédé pour traiter l'AVC chez un patient qui a eu un AVC au moins trois heures auparavant, le procédé comprenant l'administration d'au moins six millions de cellules neuronales viables à une concentration de cellules fondée sur le nombre total de cellules viables à au moins une zone cérébrale touchée par l'AVC, dans lesquelles, si lesdites cellules sont des cellules souches, elles n'ont pas été obtenues par destruction d'un embryon humain.

2. Cellules neuronales selon la revendication 1, le procédé comprenant en outre l'utilisation d'un foret hélicoïdal ou d'une fraise pour assurer la pénétration dans le crâne, les cellules pouvant ainsi être administrées.

3. Cellules neuronales selon la revendication 1 ou la revendication 2, les cellules étant choisies parmi des cellules neuronales hNT, des cellules souches neuronales, des cellules HCN-1, des cellules foetales de porc, des cellules de crête neurale ou une association de celles-ci.

4. Cellules neuronales selon l'une quelconque des revendications précédentes, l'AVC ayant eu lieu au moins trois mois auparavant.

5. Cellules neuronales à utiliser dans un procédé pour l'amélioration de la parole chez une personne ayant subi une lésion cérébrale qui interfère dans la parole, la lésion cérébrale résultant d'un AVC, le procédé comprenant l'injection d'une composition stérile d'au moins six millions de cellules neuronales viables à une concentration de cellules fondée sur le nombre total de cellules viables dans la zone endommagée, dans lesquelles, si les cellules sont des cellules souches, elles n'ont pas été obtenues par destruction d'un embryon humain.

6. Cellules neuronales selon la revendication 5, les cellules neuronales injectées étant des cellules neuronales humaines ou des cellules souches neuronales humaines.

7. Cellules neuronales à utiliser dans un procédé pour améliorer les performances motrices chez une personne qui a subi une lésion cérébrale qui interfère dans le mouvement, la lésion cérébrale résultant d'un AVC, le procédé comprenant l'injection d'une composition stérile d'au moins six millions de cellules neuronales viables à une concentration de cellules fondée sur le nombre total de cellules viables dans la zone endommagée, dans lesquelles, si les cellules sont des cellules souches, elles n'ont pas été obtenues par destruction d'un embryon humain.

8. Cellules neuronales selon la revendication 7, les cellules neuronales injectées étant des cellules neuronales humaines ou des cellules souches neuronales.

9. Cellules neuronales à utiliser avec un procédé pour améliorer la cognition chez une personne qui a subi une lésion cérébrale qui interfère dans la cognition, la lésion cérébrale résultant d'un AVC, le procédé comprenant l'injection d'une composition stérile d'au moins six millions de cellules neuronales viables à une concentration de cellules fondée sur le nombre total de cellules viables dans la zone endommagée du cerveau, dans lesquelles, si les cellules sont des cellules souches, elles n'ont pas été obtenues par destruction d'un embryon humain.

10. Cellules neuronales à utiliser dans un procédé pour améliorer la fonction sensorielle chez une personne qui a subi une lésion cérébrale qui interfère avec la sensation, la lésion cérébrale résultant d'un AVC, le procédé comprenant l'injection d'une composition stérile d'au moins six millions de cellules neuronales viables à une concentration de cellules fondée sur le nombre total de cellules viables dans la zone endommagée, dans lesquelles, si les cellules sont des cellules souches, elles n'ont pas été obtenues par destruction d'un embryon humain.

11. Cellules neuronales à utiliser dans un procédé pour améliorer la fonction sensorielle, motrice ou cognitive chez une personne qui a subi une lésion cérébrale qui interfère avec ces fonctions, la lésion cérébrale résultant d'un AVC, le procédé comprenant l'injection d'une composition stérile d'au moins six millions de cellules neuronales viables à une concentration de cellules fondée sur le nombre total de cellules viables en un emplacement à partir duquel les cellules neuronales migrent vers la zone endommagée, dans lesquelles, si les cellules sont des cellules souches, elles n'ont pas été obtenues par destruction d'un embryon humain.

12. Cellules neuronales selon la revendication 11, la composition étant administrée dans les citernes.

13. Cellules neuronales à utiliser dans un procédé pour remplacer chez une personne les nerfs du système nerveux central perdus en raison d'un AVC, le procédé comprenant l'administration à la personne d'une composition stérile d'au moins six millions de cellules neuronales viables, à une concentration de cellules fondée sur le nombre total de cellules viables, dans lesquelles, si lesdites cellules sont des cellules souches, elles n'ont pas été obtenues par destruction d'un embryon humain.

14. Cellules neuronales selon l'une quelconque des revendications 9 à 13, les cellules neuronales étant des cellules neuronales humaines ou des cellules souches neuronales.

15. Utilisation de cellules neuronales pour la fabrication d'un médicament pour traiter l'AVC chez un patient qui a subi un AVC au moins trois heures auparavant, par un procédé qui comprend l'administration d'au moins six millions de cellules neuronales viables à une concentration de cellules fondée sur le nombre total de cellules viables à au moins une zone cérébrale touchée par l'AVC, dans lesquelles, si les cellules sont des cellules souches, elles n'ont pas été obtenues par destruction d'un embryon humain.
